Europäisches Patentamt

European Patent Office

Office européen des brevets

(19)

(11) Veröffentlichungsnummer: **0 218 032**
**B1**

# (12) EUROPÄISCHE PATENTSCHRIFT

(45) Veröffentlichungstag der Patentschrift:
**01.03.89**

(21) Anmeldenummer: **86110706.8**

(22) Anmeldetag: **02.08.86**

(51) Int. Cl.⁴: **A61F 2/38**

(54) **Tibiateil für eine Kniegelenkprothese.**

(30) Priorität: **12.09.85 CH 3939/85**

(43) Veröffentlichungstag der Anmeldung:
**15.04.87 Patentblatt 87/16**

(45) Bekanntmachung des Hinweises auf die Patenterteilung:
**01.03.89 Patentblatt 89/9**

(84) Benannte Vertragsstaaten:
**AT DE FR GB IT**

(56) Entgegenhaltungen:
**EP-A- 0 151 724**
**FR-A- 2 403 068**

(73) Patentinhaber: **GEBRÜDER SULZER AKTIENGESELLSCHAFT, Zürcherstrasse 9, CH-8401 Winterthur(CH)**

(72) Erfinder: **Horber, Willi, Turbinenstrasse 12, CH-8005 Zürich(CH)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf 1(DE)**

**Beschreibung**

Die Erfindung betrifft einen Tibiateil für eine Kniegelenkprothese, in deren tibialem Plateau Verankerungszapfen für eine Verankerung im Knochen vorgesehen sind, während die dem Plateau gegenüberliegende Oberfläche als tibiale Gleitfläche ausgebildet ist, wobei jeder Verankerungszapfen von mindestens einer, zu seiner Achse schrägflächigen und konzentrischen Kegelstumpffläche umgeben ist.

Ein Tibiateil der vorstehend genannten Art ist aus der EP-A 0 151 724 bekannt. In der Praxis hat sich nun gezeigt, dass die Gleitflächen der Gelenkpfannen auf der Tibia häufig in stark unterschiedlichem Masse geschädigt sind, d.h. dass beispielsweise die laterale Gelenkpfanne stärker angegriffen ist als die mediale oder umgekehrt. Bei der bisherigen Konstruktion richtet sich die erforderliche Knochenresektion nach der "Tiefe" der stärksten Zerstörung; dabei muss im Bereich geringerer Beschädigungen unnötigerweise gesunde Knochensubstanz entfernt werden.

Aufgabe der Erfindung ist es, diesen unnötigen Verlust an Knochensubstanz im natürlichen Tibia-Plateau zu vermindern oder ganz zu vermeiden.

Diese Aufgabe wird mit der vorliegenden Erfindung dadurch gelöst, dass die schrägflächigen Kegelstumpfflächen für die einzelnen Verankerungszapfen von einer Bezugsebene des Tibia-Plateaus einen unterschiedlichen Abstand haben.

Bei einem derart ausgebildeten Tibiateil ist es möglich, in der Tibia für die Kegelstumpfflächen im Bereich des lateralen bzw. des medialen Verankerungszapfens – je nach den medizinischen Erfordernissen – unterschiedlich tief auszufräsen; damit ist es nicht mehr notwendig, bei unterschiedlichen lateralen bzw. medialen Schädigungen gegebenenfalls gesundes Knochengewebe der Tibia unnötig zu resezieren.

Eine besonders einfache Fabrikation des neuen Tibiateiles ergibt sich, wenn er aus einem Plattenkörper und einer schalenartigen, den Plattenkörper aufnehmenden Tibiaunterlage besteht, wobei zusätzlich die Massnahme getroffen sein kann, dass die Tibiaunterlage aus mit einem Verankerungszapfen versehenen konzentrischen kreiszylindrischen Kegelstumpfkörpern besteht, die durch Sekantenschnitte parallel zu der Zapfenachse mit ebenen Anlageflächen versehen sind, längs denen sie miteinander verschweisst sind.

Wegen der günstigen Gleiteigenschaften besteht der neue Tibiateil vorzugsweise mindestens teilweise aus Kunststoff, z.B. aus Polyäthylen; es ist jedoch auch möglich, den neuen Tibiateil aus einem beliebigen anderen der bekannten Implantat-Werkstoffe – wie Metalle, Metall-Legierungen, biokeramische Werkstoffe, Kohlenstoff oder faserverstärkte Verbundwerkstoffe – herzustellen oder ihn aus zwei oder mehr dieser Werkstoffe – insbesondere aus einem Metall- und einem Kunststoffteil – zusammenzusetzen. Weiterhin können seine Oberflächen ganz oder teilweise mit bioinerten oder bioaktiven Beschichtungen versehen sein.

Im folgenden wird die Erfindung anhand eines Ausführungsbeispiels im Zusammenhang mit der Zeichnung näher erläutert.

Fig. 1 ist – teilweise im Schnitt I–I von Fig. 2 – eine Anterior/posterior-Ansicht des neuen Tibiateils;

Fig. 2 gibt eine Ansicht von Fig. 1 von unten wieder.

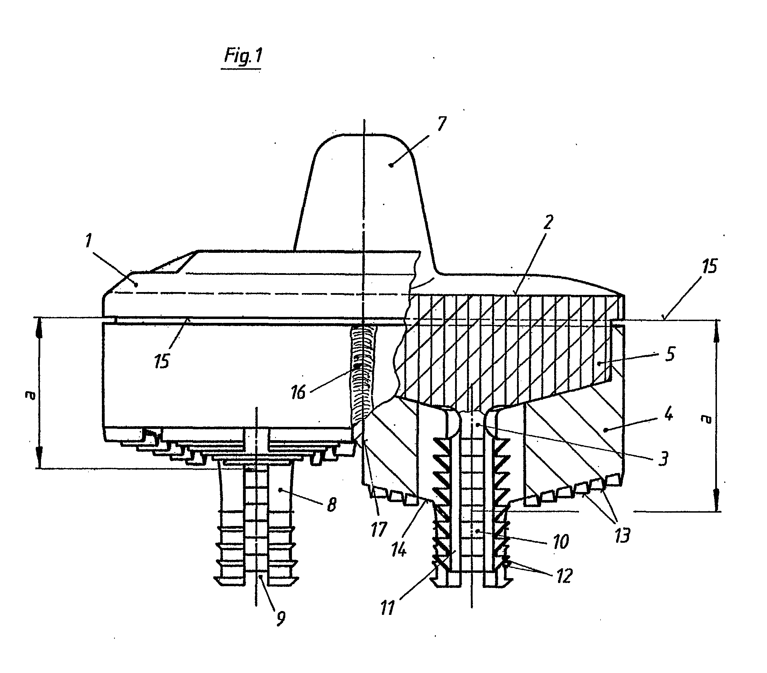

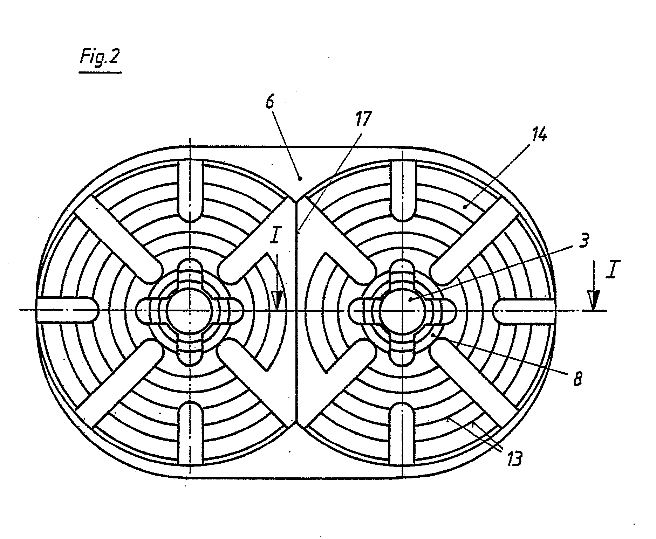

Der als Ausführungsbeispiel gezeigte Tibiateil ist eine aus Metall und Kunststoff bestehende zweiteilige Konstruktion; ein Plattenkörper 1 (Fig. 1) aus Kunststoff enthält die tibialen Gleitflächen oder Gelenkpfannen 2 und ist in einer trog- oder schalenartigen Vertiefung einer metallenen Tibiaunterlage 4 gelagert. An sein dem Knochen bzw. der Unterlage 4 zugewandtes Tibia-Plateau 6 (Fig. 2) sind kreiszylindrische Ansätze 5 angesetzt, die über einen konisch verlaufenden Boden in einem Verankerungsschaft 3 enden.

Die Verankerungszapfen 3 bilden die Spreizkörper einer bereits früher beschriebenen dübelartigen Verankerung. Bei dieser zementfreien Verankerung dringt der Spreizkörper in einen Hohlkörper 8 ein, der im vorliegenden Fall den Verankerungszapfen der Tibiaunterlage 4 bildet; dabei weitet der Spreizkörper dessen durch Längsschlitze 9 in einzelne Stege aufgelösten Mantel auf. Der Spreizkörper bildet dabei ein "Negativ" des Hohlkörpers 8, wobei er an die Längsschlitze 9 angepasste Vorsprünge 10 und an die Mantelstege angepasste Längsausnehmungen 11 hat. Vorsprünge und Stege sind mit widerhakenartigen Rippen 12 versehen.

Beim implantierten Tibiateil bilden Mantelstege und Vorsprünge auf diese Weise einen "geschlossenen Zapfen", an den und in den Gewebe allseitig gleichmässig einwachsen kann.

Auf den Gleitflächen oder Gelenkpfannen 2 können kondylenähnlich geformte Gegenflächen des nicht gezeigten Femurteils gleitend abrollen. Zwischen den Gleitflächen 2 erhebt sich nach posterior versetzt ein Führungszapfen 7, durch den der Femurteil bei seiner gleitenden Abrollbewegung geführt ist.

Die Tibiaunterlage 4 besteht aus zwei in ihren trogartigen Vertiefungen die Ansätze 5 aufnehmenden kreiszylindrischen Metallschalen. Diese Böden sind durch sekantenartige Schnitte (Fig. 2) parallel zu den Achsen der Verankerungszapfen 3, 8 mit ebenen Auflageflächen 17 versehen, längs denen sie zusammengefügt und miteinander verschweisst sind (Schweissnähte 16). Den äusseren Abschluss der Zylinderschalen nach unten bilden wiederum konische Kegelstumpfflächen 14, die zentral in den Verankerungszapfen oder Hohlkörpern 8 enden und mit schneidenartigen Rippen 13 bedeckt sind.

Erfindungsgemäss haben die Kegelstumpfflächen 14 für den in Fig. 1 rechten Verankerungszapfen 3, 8 einen willkürlich festzulegenden, jedoch eindeutig grösseren Abstand a von einer Bezugsebene 15 des Tibia-Plateaus 6, die beispielsweise durch die Unterkante dieses Plateaus oder durch eine Oberkante der metallenen Unterlage 4 gebildet sein kann, als die entsprechenden Kegelstumpfflächen für den linken Verankerungszapfen.

## Patentansprüche

1. Tibiateil für eine Kniegelenkprothese, in deren tibialem Plateau (6) Verankerungszapfen (3, 8) für eine Verankerung im Knochen vorgesehen sind, während die dem Plateau gegenüberliegende Oberfläche als tibiale Gleitfläche (2) ausgebildet ist, wobei jeder Verankerungszapfen (3, 8) von mindestens einer, zu seiner Achse schrägflächigen und konzentrischen Kegelstumpffläche (14) umgeben ist, dadurch gekennzeichnet, dass die schrägflächigen Kegelstumpfflächen (14) für die einzelnen Verankerungszapfen (3, 8) von einer Bezugsebene (15) des Tibia-Plateaus (6) einen unterschiedlichen Abstand (a) haben.

2. Tibiateil nach Anspruch 1, dadurch gekennzeichnet, dass er aus einem Plattenkörper (1) und einer schalenartigen den Plattenkörper (1) aufnehmenden Tibiaunterlage (4) besteht.

3. Tibiateil nach Anspruch 1 oder 2, dadurch gekennzeichnet, dass die Tibiaunterlage (4) aus mit einem Verankerungszapfen (8) versehenen konzentrischen kreiszylindrischen Kegelstumpfkörpern besteht, die durch Sekantenschnitte parallel zu der Zapfenachse mit ebenen Anlageflächen (17) versehen sind, längs denen sie miteinander verschweisst (16) sind.

## Claims

1. A tibia part for a knee-joint prosthesis having a tibial plateau (6) comprising anchoring pins (3, 8) for anchoring in the bone, whereas the surface opposite the plateau is constructed as a tibial sliding surface (2), each anchoring pin (3, 8) being surrounded by at least one frusto-conical surface (14) which is concentric with and at an angle to the pin axis, characterised in that the oblique frusto-conical surfaces (14) for the individual anchoring pins (3, 8) are at different distances (a) from a reference plane (15) of the tibia plateau (6).

2. A tibia part according to claim 1, characterised in that it comprises a flat member (1) and a dish-like tibia base (4) receiving the flat member (1).

3. A tibia part according to claim 1 or 2, characterised in that the tibia base (4) comprises concentric circular cylindrical frusto-conical members comprising an anchoring pin (8) and, by means of secant cuts parallel to the pin axis, provided with flat bearing surfaces (17) along which they are welded (16) together.

## Revendications

1. Elément de tibia pour une prothèse de l'articulation du genou dans le plateau de tibia (6) duquel sont prévues des tiges d'ancrage (3, 8) destinées à un ancrage dans l'os, tandis que la surface opposée au plateau sert de surface de glissement (2) du tibia, chaque tige d'ancrage (3, 8) étant entourée par au moins une surface tronconique (14) oblique par rapport à son axe et concentrique, caractérisé en ce que les surfaces tronconiques (14) obliques des différentes tiges d'ancrage (3, 8) sont à une distance (a) différente d'un plan de référence (15) du plateau de tibia (6).

2. Elément de tibia selon la revendication 1, caractérisé en ce qu'il est constitué d'un corps en plaque (1) et d'un support de tibia (4) en forme de coque, dans lequel est logé le corps en plaque (1).

3. Elément de tibia selon la revendication 1 ou 2, caractérisé en ce que le support de tibia (4) est constitué de corps tronconiques cylindriques circulaires, concentriques, pourvus d'une tige d'ancrage (8), ces corps tronconiques étant pourvus, par des entailles sécantes parallèles à l'axe de la tige, de surfaces de butée (17) planes le long desquelles ils sont soudés (16) entre eux.

*Fig. 1*

Fig.2